# EUROPEAN PATENT APPLICATION

(11) **EP 4 529 846 A1**
(43) Date of publication of application: **02.04.2025**
(21) Application number: 24199978.8
(22) Date of filing: 12.09.2024
(51) Int. Cl.: A61B 6/00

(54) **DEVICE AND METHOD FOR INTEGRATED TISSUE SAMPLE IMAGING ON RADIOGRAPHY IMAGING SYSTEM**

(30) Priority: 29.09.2023 US 202318374808
(71) Applicant: GE Precision Healthcare LLC, Waukesha, WI 53188 (US)
(72) Inventor: MILIONI DE CARVALHO, Pablo, 92370 Chaville (FR); VANCAMBERG, Laurence, 78300 Poissy (FR); CARTON, Ann-Katherine, 92140 Clamart (FR)
(74) Representative: Kilburn & Strode LLP

(57) **Abstract**

A tissue imaging system (300) for use in conjunction with a radiography imaging system (10) including a radiation source (16) and a detector (18) includes a tissue imaging device (302) adapted to be positioned on a surface (50) of the detector (18) of the radiography imaging system (10) between the radiation source (16) and the detector (18). The tissue imaging device (302) includes a housing (314) positionable on the surface (50) of the detector (18), the housing (314) defining an interior and a tissue support surface (319) opposite the surface (50) of the detector (18) and a photon counting energy discriminating (PCED) detector (316) disposed within the interior of the housing (314). The PCED detector (316) is removably connected to the radiography imaging system (10) and is operable to generate image data used to form one or more of multi-energy material decomposition images, material enhanced images, 2D/3D image tomographic reconstruction images, and phase contrast images of the imaged tissue positioned on the tissue support surface (319).

## Description

### FIELD OF THE DISCLOSURE

The present disclosure relates generally to medical imaging systems, including mammography systems and devices, and more specifically to biopsy sample imaging systems utilized with a medical imaging system.

### BACKGROUND OF THE DISCLOSURE

Embodiments of the invention relate generally to X-ray medical imaging, and more particularly to imaging systems or devices and methods employed to perform various imaging procedures, such as mammography imaging procedures including but not limited to spectral mammography (SM), such as 2D/3D dual-energy contrast-enhanced (CE) mammography exams, full-field digital mammography (FFDM) or digital breast tomosynthesis (DBT) mammography exams.

Spectral mammography (SM) is an X-ray imaging modality used to scan breasts for screening, diagnosis and/or interventional examinations. The effectiveness of spectral mammography is affected by numerous factors, one of which is the two-dimensional (2D) rendering of images obtained using SM.

Alternative systems to SM are also known for breast imaging. Some examples include full-field digital mammography, which captures the image directly onto a flat-panel detector, computed radiography, which involves the use of a cassette that contains an imaging plate), or digital breast tomosynthesis (DBT). A digital breast tomosynthesis (DBT) or mammography-tomography (mammo-tomo) system is a dedicated mammography system that acquires several (e.g., tens of) angularly offset projection X-ray images and uses the resulting X-ray image data to reconstruct three-dimensional (3D) image datasets.

The 3D image datasets are used to form various volumetric representations of the imaged breast, including an entire 3D volume of the breast, and various 3D sections of the 3D volume, such as slices or slabs constituting specified thicknesses of the 3D volume oriented to provide the desired view of one or more regions of interest (ROI) detected within the 3D image dataset.

In addition, when the 3D image datasets of the breast have been produced, after being utilized in a suitable imaging/diagnosis procedure, they can be utilized to guide a biopsy device employed with the DBT system into the breast to obtain a biopsy of the region of interest (ROI) identified within the 3D image datasets. In DBT systems, the biopsy device is disposed directly on the DBT system in order to be able to perform the biopsy utilizing the 3D image dataset to guide the biopsy device to the ROI. The mammography imaging systems provide high quality diagnostic mammographic images of breast tissue to enable any ROIs therein to be identified and sampled for further analysis in a biopsy procedure performed subsequent to the imaging/diagnosis process.

However, the quality of the images provided by the mammography imaging system, while suitable for initial location and diagnosis of the ROIs, is not suitable for use in the more precise classification of the tissue forming and/or present within the ROIs, e.g., microcalcifications. For this purpose, more detailed images of the biopsy sample(s) obtained from the ROIs need to be generated. On many occasions, the biopsy samples are sent to a histopathologist for use in separate imaging system to obtain imaging information concerning the type of tissue(s) present within the biopsy sample(s). The histopathology imaging system can be any of a number of types of imaging system(s), including by high resolution optical systems, i.e., a digital microscope, with a suitable contrast agent(s) applied to the biopsy sample, or x-ray based imaging systems similar to those discussed previously, but configured for high-resolution images to discriminate between tissue types in the biopsy sample(s), phase contrast x-ray imaging systems, and/or x-ray systems including photon counting energy discrimination (PCED) detectors.

While capable of providing the necessary information regarding the types of tissue(s) present within the biopsy samples, the time required to send the biopsy samples to the location of the histopathology imaging system and to obtain the images illustrating the types of tissue(s) present in the biopsy samples is undesirable. More particularly, the delay caused by sending the biopsy samples to a separate location and/or imaging system for can present issues regarding the timeliness of the histopathology results being provided to the physician and/or the patient.

In one prior art attempt to address this issue, as shown in FIG. 1 US Patent No. 9,603,577, entitled *X-Ray Imaging Apparatus And Control Method Thereof,* (the `577 Patent) the entirety of which is expressly incorporated herein for all purposes, discloses an x-ray imaging apparatus 1000 including an x-ray source 1100, and a pair of detectors 1200A,1200B disposed moveably disposed relative to the x-ray source 1100. The first detector 1200A is laterally moveable in order to selectively position the first detector 1200A below an object OB to be imaged and in the path of x-rays emitted from the x-ray source 1100 that pass through the object. The second detector 1200B is disposed below the first detector 1200A and is vertically moveable relative to the object and x-ray source 110 to position a collimator 1300 attached to the second detector 1200B within the path of x-rays emitted from the source. When the first detector 1200A is laterally moved away from the object, e.g., retracted within the housing 1020 of the imaging apparatus 1000, the first detector 1200A is disposed out of the path of the emitted x-rays, which can pass through the object and contact the second detector 1200B. With this configuration for the x-ray imaging apparatus 1000, both adsorption and phase contrast images of the object can be obtained.

However, the integration of the first detector 1200A and the second detector 1200B operably into the structure of the imaging apparatus 1000 requires a high degree of complexity for the structure and manner of operation of the imaging apparatus of the ` 577 Patent, making the imaging apparatus 1000 disclosed therein less than ideal for the purposes of providing diagnostic and histopathology images with a single imaging system.

Therefore, with regard to the aforementioned shortcomings of prior art imaging systems concerning the ability of those imaging systems to obtain both diagnostic images for use in biopsy procedures and histopathologic images of the obtained biopsy samples, it is desirable to develop an improved imaging system and method for obtaining both diagnostic and histopathologic images on a single imaging system.

### SUMMARY OF THE DISCLOSURE

According to one aspect of an exemplary embodiment of the present disclosure, a tissue imaging system for use in conjunction with a radiography imaging system including a radiation source and a detector includes a tissue imaging device adapted to be positioned on a surface of the detector of the radiography imaging system between the radiation source and the detector, the tissue imaging device having a housing positionable on the surface of the detector, the housing defining an interior and a tissue support surface opposite the surface of the detector; and a photon counting energy discriminating (PCED) detector disposed within the interior of the housing, the PCED detector adapted to generate image data used to form one or more of multi-energy material decomposition images, material enhanced images, 2D/3D image tomographic reconstruction images, and phase contrast images of the imaged tissue.

According to still another aspect of an exemplary embodiment of the present disclosure, a radiography imaging system includes a radiation source, a detector capable of receiving radiation from the radiation source and defining a surface, a controller operable to control the operation and movement of the radiation source and detector to generate image data, the controller including a central processing unit and interconnected memory for processing the image data from the detector, a display operably connected to the controller for presenting information to a user, a user interface operably connected to the controller to enable user input to the controller, and a tissue imaging system having a housing removably positionable on the surface of the detector, the housing defining an interior and a tissue support surface opposite the surface of the detector and a photon counting energy discriminating (PCED) detector disposed within the interior of the housing, the PCED detector removably connected to the controller and adapted to generate image data of the imaged tissue to form one or more of multi-energy material decomposition images, material enhanced images, 2D/3D image tomographic reconstruction images, and phase contrast images of the imaged tissue.

According to still another aspect of an exemplary embodiment of the present disclosure, a method for generating image data for imaged tissue to produce one or more of multi-energy material decomposition images, material enhanced images, 2D/3D image tomographic reconstruction images, and phase contrast images of the imaged tissue on a radiography imaging system includes the steps of providing a radiography imaging system including a radiation source, a detector capable of receiving radiation from the radiation source and defining a surface, a controller operable to control the operation and movement of the radiation source and detector to generate image data, the controller including a central processing unit and interconnected memory for processing the image data from the detector, a display operably connected to the controller for presenting information to a user, and a user interface operably connected to the controller to enable user input to the controller, positioning a tissue imaging device on the surface of the detector, the tissue imaging device having a housing defining an interior and a tissue support surface opposite the surface of the detector, and a photon counting energy discriminating (PCED) detector disposed within the interior of the housing and removably connected to the controller, positioning the imaged tissue on the tissue support surface between the radiation source and the PCED detector, and operating the radiation source to direct radiation through the imaged tissue into contact with the PCED detector and generate the image data for the imaged tissue.

These and other exemplary aspects, features and advantages of the invention will be made apparent from the following detailed description taken together with the drawing figures.

### BRIEF DESCRIPTION OF THE DRAWINGS

The drawings illustrate the best mode currently contemplated of practicing the present invention.

In the drawings:

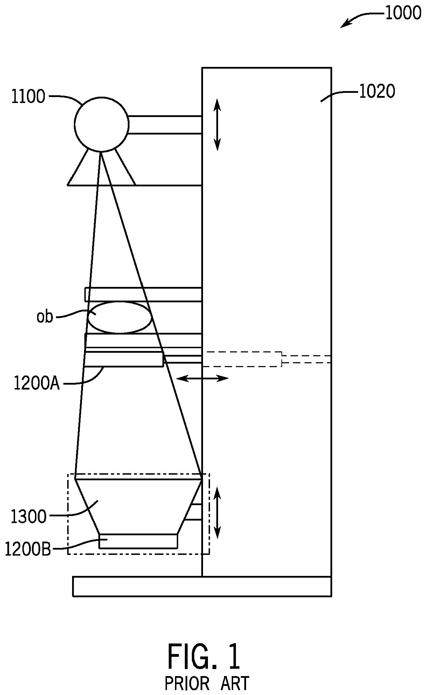

FIG. 1 is an isometric view of a prior art phase contrast mammography imaging system.
FIG. 2 is a perspective view of an exemplary mammography imaging system in accordance with an embodiment of the disclosure.
FIG. 3 is a diagram of the mammography imaging system of FIG. 2, showing the radiation source of the system in a scanning position, in accordance with an embodiment of the disclosure.
FIG. 4 is a side elevation view of the mammography imaging system of FIG. 2 including a biopsy system thereon in accordance with an embodiment of the disclosure.
FIG. 5 is a side elevation view of the mammography imaging system of FIG. 4 including a biopsy imaging system thereon, in accordance with an embodiment of the disclosure.
FIG. 6 is a schematic view of the mammography imaging system and biopsy imaging system of FIG. 5, in accordance with an embodiment of the disclosure.
FIG. 7 is a schematic view of the mammography imaging system and biopsy imaging system of FIG. 5, in accordance with another embodiment of the disclosure.
FIG. 8 is a side elevational view of the mammography imaging and biopsy imaging system of FIG. 5, in accordance with still another embodiment of the disclosure.
FIG. 9 is a schematic view of the mammography imaging system and biopsy imaging system of FIG. 5, in accordance with another embodiment of the disclosure.
FIG. 10 is a schematic view of an image displayed by the mammography imaging system and biopsy imaging system of FIG. 5, in accordance with another embodiment of the disclosure.

### DETAILED DESCRIPTION OF THE DRAWINGS

One or more specific embodiments will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

As used herein, "electrically coupled", "electrically connected", and "electrical communication" mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present.

Further, while the embodiments disclosed herein are described with respect to a mammography apparatus for the 2-dimensional imaging of breast tissue, it is to be understood that embodiments of the invention may be applicable to other types of imaging systems for both 2-dimensional and 3-dimensional imaging including, for example, fluoroscopy, full-filed digital mammography, digital breast tomosynthesis (DBT) and spectral mammography (single or multi-energy), as well as for imaging procedures for tissue other than breast tissue. Further still, embodiments of the invention may be used to analyze tissue, generally, and are not limited to analyzing human tissue.

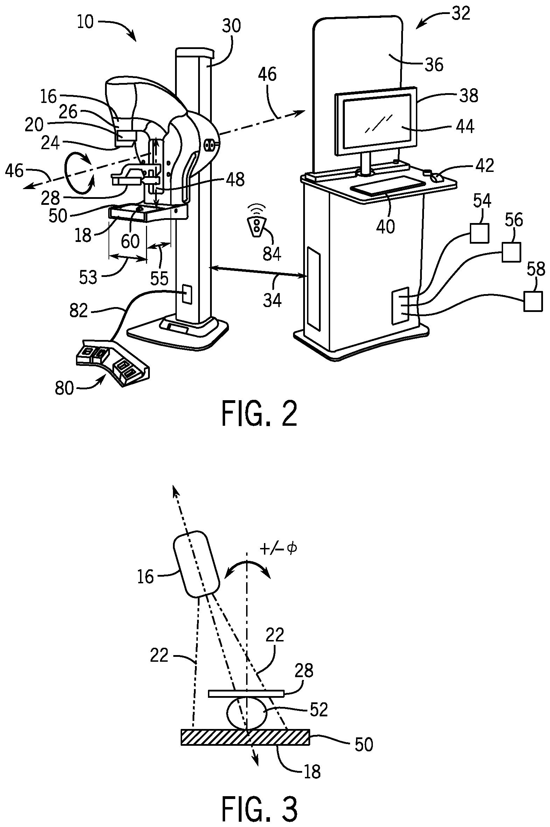

Referring now to FIGS. 2 and 3, the major components of an exemplary radiography imaging system 10 formed as a mammography imaging system 12 for imaging breast tissue according to one embodiment of the disclosure are shown. The radiography imaging system 10, such that disclosed in US Patent Application Publication No. US20200060632, entitled *Apparatus And Method For Mammographic Breast Compression,* the entirety of which is expressly incorporated herein by reference for all purposes, includes a radiation source/x-ray source 16, a radiation detector 18, and a collimator 20. The radiation source 16 is movable between a variety of imaging positions relative to the detector 18, and is operative to emit radiation rays 22 (FIG. 2) that are received by the radiation detector 18 to provide an image of an object, such as a breast 52. The radiation source(s) 16 can be operated to emit x-rays at one or more energy spectra, e.g., at low energy (a source operating voltage of about 60-80 kVp) and a high energy (a source operating voltage of about 120-140 kVp) and in other embodiments may emit at polychromatic spectra localized around energy levels (i.e., spectra comprising specific kVp ranges) other than those listed herein and/or at more than two emission spectra for a given examination. Selection of the respective energy levels for emission may be based, at least in part, on the anatomy being imaged and the materials of interest for tissue characterization. In embodiments, the radiography imaging system 10 may include a patient shield 24 mounted to the radiation source 16 via face shield rails 26 to prevent the patient's head from obstructing the radiation rays and protecting the patient from the radiation rays 22.

Referring still further to FIGS. 2 and 3, the radiography imaging system 10 also includes a compression paddle or plate 28 and a support structure 30 to which one or more of the radiation source 16, radiation detector 18, and/or compression plate 28 may be mounted to. In embodiments, the radiography imaging system 10 may further include a controller 32. The controller 32 may be a workstation having at least one processor/central processing unit/computer and a memory device/database that stores information and/or instructions for the operation of the radiography system 10 that are employed by the controller 32, as shown in FIG. 2 or, in other embodiments, the controller 32 may be embedded/integrated into one or more of the various components of the system 10 disclosed above. In embodiments, the controller 32 may be in electrical communication with the radiation source 16, radiation detector 18, and/or the compression plate 28 via a cable 34. As will be appreciated, in embodiments, the connection 34 may be a wireless connection. In embodiments, the controller 32 may include a radiation shield 36 that protects an operator of the radiography imaging system 10 from the radiation rays 22 emitted by the radiation source 16. The controller 32 may further include a display 38, a keyboard 40, mouse 42, and/or other appropriate user input devices that facilitate control of the radiography imaging system 10 via a user interface 44.

As further shown in FIGS. 2 and 3, the radiation source 16, along with the radiation detector 18, forms part of an x-ray system which provides x-ray imagery for the purpose of imaging a body part of a patient, such as breast 52. As stated above, the radiation source 16 emits the radiation rays 22 such that the radiation rays 22 travel from the radiation source 16 to the radiation detector 18. While the radiation rays 22 are discussed herein as being x-rays, it is to be understood that the radiation source 16 may emit other types of electromagnetic rays which can be used to image a patient. The radiation source 16 may be mounted to the support structure 30 such that the radiation source can rotate around an axis 46 in relation to the radiation detector 18, although movement of the radiation source 16 in paths other than rotation about a fixed axis, such as during digital breast tomosynthesis (DBT), are also envisioned. In embodiments, the radiation detector 18 may be configured to rotate or translate within its housing, such as in the directions indicated by arrows 53 and 55.

In the illustrated exemplary embodiment of FIG. 2 the radiation source 16 and the detector 18 are mounted to a gantry 90 that is secured to the support structure 30. The support structure 30 houses a translation mechanism 92 that is operably connected to the gantry 90. The translation mechanism 92 is operable to move the gantry 90 vertically with respect to the support structure 30 in order to position the gantry 90 at the appropriate height to accommodate the dimensions of the patient on which the radiography imaging system 10 is being utilized. The translation mechanism 92 is also operable to rotate the gantry 90 relative to the support structure 30 about the horizontal axis 46 in order to position the gantry 90 rotationally with regard to the patient, as necessary.

The gantry 90 includes a generally C-shaped body 94 with the radiation source 16 at one end and the detector 18 at the opposite end. In this configuration, regardless of the vertical and/or rotational orientation of the gantry 90, such as to position the radiation source 16 and detector 18 relative to the patient breast 52 to obtain x-ray images at various orientations, such as for craniocaudal (CC) or mediolateral oblique (MLO) views, among others, the radiation source 16 is disposed in alignment with the detector 18. In this position, the detector 18 is capable of receiving the x-rays 22 emitted from the radiation source 16 that pass through the portion of the patient, i.e., patient breast 52, located between the radiation source 16 and the detector 18 in order to generate image data for transmission to the control system 32 of the radiography imaging system 10 to create/reconstruct a 3D image dataset for viewing by a physician, such as by using DBT, among other known methods.

Additionally, in another embodiment the radiation source 16 can be attached to the gantry 90 to rotate and/or move independently of the gantry 90 and detector 18 in order to enable the radiation source 16 to take x-ray images of the patient breast at various angles relative to the detector 18, e.g., between +/- 60°. The images obtained between these angles for the radiation source 16 can be used either for creation of stereoscopic images in a biopsy procedure using the system 10 or for DBT when operating the radiography imaging system 10 in an imaging mode.

As stated above, the radiation detector 18 receives the radiation rays 22 emitted by the radiation source 16. In embodiments, data regarding the radiation rays 22 received by the radiation detector 18 may be electrically communicated to the controller 32 from the radiation detector 18 via cable/electronic connection 34 such that the controller 32 generates one or more images which may be shown on the display 38 and stored in the memory device.

The compression plate 28 is operative, in response to instruction from the controller 32 or in response to instructions from controller(s) on or near the radiography imaging system 10 or switch controllers 80, to move towards and away from the radiation detector 18 as indicated by arrows/compression axis 48 such that the compression plate 28 flattens and holds a body part, e.g., breast 52, in place against the surface 50 of the radiation detector 18. In this respect, the radiation detector 18 and the surface 50 thereof is referred to herein as a "compression surface or support plate" that cooperates with the compression plate 28 to compress and clamp a breast of a patient therebetween.

In one exemplary embodiment, in order to maintain the position of the patient breast 52 stationary during the imaging and/or biopsy procedures, the compression plate 28 is attached to a plate or paddle support mechanism 45 located on and/or within the gantry 90 that positions the compression plate 28 directly over and in alignment with the detector 18/compression surface 50 and operably connected to the controller 32. The plate support mechanism 45 is operable within the gantry 90 at any rotational or vertical position of the gantry 90 to move the plate 28 in a line either towards or away from the detector 18/ compression surface 50. The mechanism 45 can have any of a number of different configurations, but in one exemplary embodiment takes the form of a compression screw mechanism that is operable to move the plate 28 into engagement with the patient breast 52 to exert a predetermined pressure/compression on the breast 52 to retain the breast 52 in a stationary position between the plate 28 and the detector 18/compression surface 50 during imaging and/or biopsy procedures.

In operation, in accordance with an embodiment, the breast 52 of the patient may be placed onto the compression surface 50 of the radiation detector 18. The compression plate 28, under control of the plate support mechanism 45 by the controller 32, moves towards the detector 18 to compress the breast 52 against the surface 50 of the detector 18 such that the breast 52 is immobilized. Movement of the compression plate 28 towards the detector 18 to compress the breast 52 against the detector 18/compression surface 50 defines a compression phase of the radiography imaging system 10. Once a target compression is achieved, movement of the compression plate 28 is halted and the compression plate 28 and the detector 18/compression surface 50 are held in fixed position to clamp the breast 52 therebetween (referred to herein as the clamping phase) so that imaging or procedures, e.g., a biopsy, may be commenced. During an imaging procedure, the radiation source 16 is selectively adjusted such that it is moved/rotated to a first scanning position and scans the breast 52. The radiation detector 18 receives the radiation rays 22 passing through the breast 52 and sends data to the controller 32 which then generates one or more x-ray images of the breast 52. Once imaging is complete, the controller 32 moves the compression plate 28 away from the detector 18/compression surface 50 to free the breast 52.

Referring still further to FIG. 2, in an embodiment, the radiography imaging system 10 may include one or more physiological monitoring or sensor devices 54, 56, 58, 60 communicatively coupled with the controller 32 for monitoring one or more physiological parameters of a patient (and for transmitting physiological parameter data to the controller 32). While FIG. 2 illustrates that the sensor devices 54, 56, 58 are connected to the controller 32, in some embodiments, one or more of the sensor devices may be communicatively coupled with the mammography apparatus, without departing from the broader aspects of the invention. The sensor devices may be selected to monitor and/or measure any physiological information of a patient desired, including, but not limited to, diastolic blood pressure, systolic blood pressure, body temperature, blood oxygen level, patient weight, skin conductance, pulse rate, etc. As illustrated in FIG. 2, one or more of the sensor devices, e.g., sensor device 60, may be physically integrated with the compression plate 28 and/or the detector 18/compression surface 50. By incorporating the sensor devices into the detector 18/compression surface 50 or compression plate 28, physiological parameter data of the patient may be acquired and transmitted to the controller 32 without requiring any additional intervention by the system operator.

In an embodiment, the sensor device 60 may be a force sensor for measuring the amount of pressure or compressive force applied to the breast 52. Additional sensors for measuring physiological parameters may be configured to either directly measure or allow the calculation of variables such as force, pressure, temperature, rigidity, elasticity, breast size and/or volume, and/or tissue density and could be embedded in compression plate 28 or detector 18/compression surface 50 or attached as part of radiography imaging system 10.

Referring once again to FIG. 2, in an embodiment, operation of the radiography imaging system 10 during the compression phase and the clamping phase may be controlled by the patient using switch controls 80, e.g., footswitch controls. Switch controls 80 are typically connected via a cable/wire 82 to radiography imaging system 10. The controls are also often mirrored on the opposite side of radiography imaging system 10 (not shown). Other controls (not shown) may be present on particular accessories placed either in the paddle/breast support area. In an embodiment, rather than being footswitch controls, the switch controls may be a handheld control unit 84 with a wired, wireless, Bluetooth or other connection with the radiography imaging system 10.

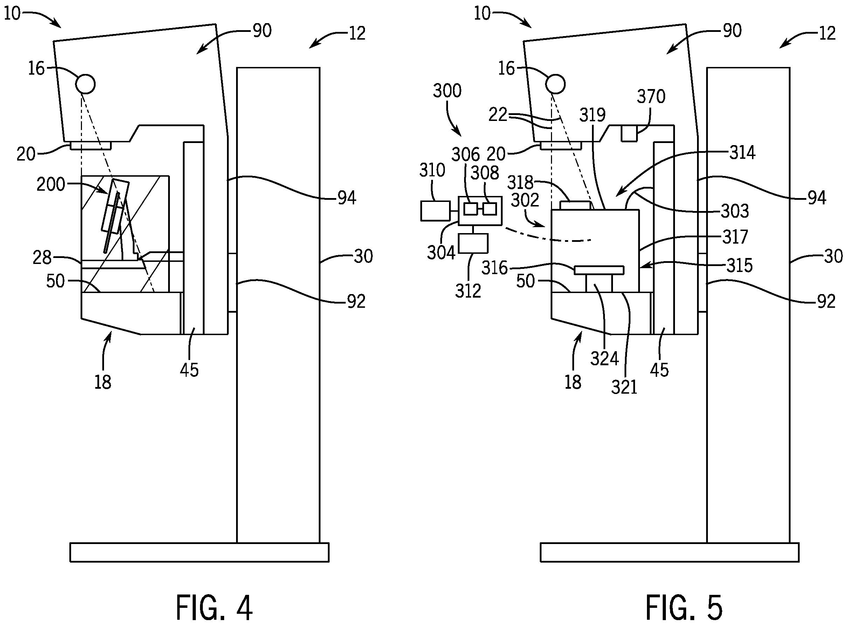

Referring now to FIG. 4, the radiography imaging system 10 may further, or alternatively, include a biopsy system 200, which may be selectively removable from the radiography imaging system 10. In such an embodiment, the radiation source 16, along with the radiation detector 18, forms part of an x-ray system which provides x-ray imagery for the purpose of guiding the biopsy system 200 to a suspect site within a body part of a patient. As shown in FIG. 4, in embodiments, the biopsy system 200, may be disposed on the support structure 30 such that it also rotates about the axis 46, in a manner similar to the radiation source 16, and/or moves in a vertical and/or horizontal direction, in a manner similar to the compression plate 28.

Looking now at the exemplary illustrated embodiments of FIGS. 5-7, the radiography imaging system 10, with or without the biopsy system 200, includes a tissue or biopsy imaging system 300 operably disposed on the radiography imaging system 10. The biopsy imaging system 300 includes a tissue or biopsy imaging device 302 that is positioned directly on the surface 50 over the detector 18 and a control device 304 operably connected to the biopsy imaging device 302. The biopsy imaging device 302, which is also operably connected to the radiography imaging system 10, such as to the gantry 90 using a direct wired connection 303 for communication between the radiography imaging system 10 and the biopsy imaging device 302, is disposed on the surface 50 over the detector 18 optionally with the compression paddle 28 and the biopsy system 200 removed from the radiography imaging system 10.

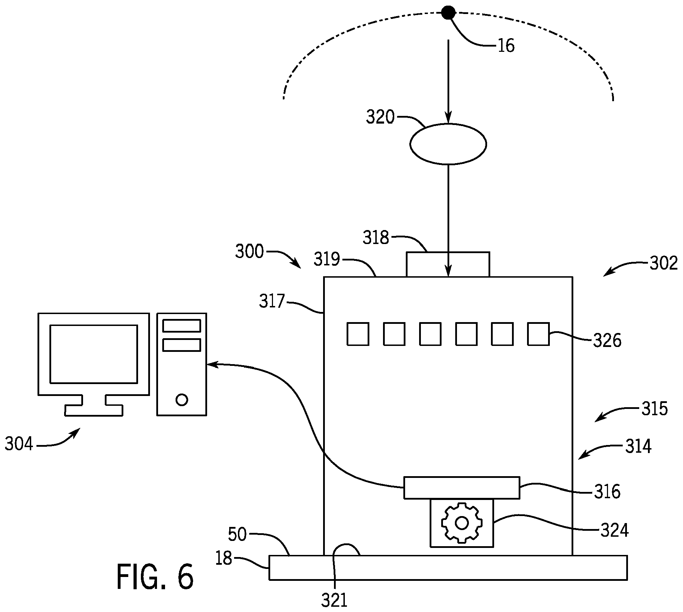

In the illustrated exemplary embodiment of FIGS. 5 and 6, the control device 304 is separate from the controller 32 and shown as including a control processor 306, an electronic memory 308 for storing operating instructions for the control processor 306, among other information, a user interface 310 and a display 312, that is operably connected to the biopsy imaging device 302, such as by a wired or wireless connection. However, embodiments where the control device 304 and/or control processor 306 acts in conjunction with other control devices, e.g., controller 32 or other control circuitry local or remote to the radiography imaging system 10, are also encompassed by the present disclosure, such that the function(s) of the control device 304 can optionally in part or in their entirety be performed by the controller 32, such that the control device 304 is not required in conjunction with the biopsy imaging device 302, with the biopsy imaging device 302 being operably connected directly to the controller 32.

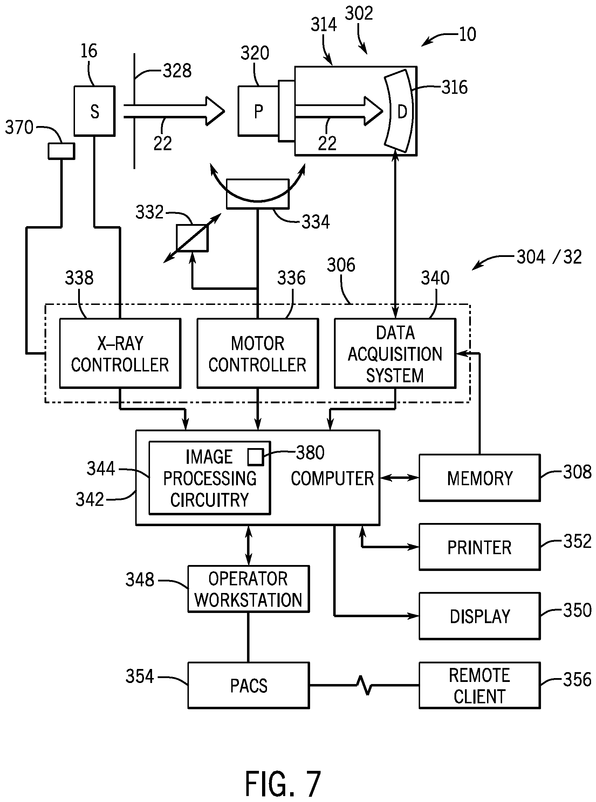

The control processor 306 commands operation of the radiography imaging system 10 and the biopsy imaging device 302 to execute filtration, examination and/or calibration protocols and may process the acquired data. With respect to the radiation source 16, the control device 304, optionally in coordination with the controller 32, furnishes power, focal spot location, control signals and so forth, for the X-ray examination sequences. In accordance with certain embodiments, the control device 304 may control operation of a filter assembly 328 (FIG. 7), the gantry 90 (or other structural support to which the radiation source 16 and detector 316 are attached), and/or the translation and/or inclination of the patient support over the course of an examination.

In addition, as shown in the illustrated embodiment of FIG. 7, the control processor 306, via a motor controller 336, may control operation of a linear positioning subsystem 332 and/or a rotational subsystem 334 used to move components of the radiography imaging system 10, biopsy imaging device 302 and/or the tissue sample 320. The control processor 306 may include signal processing circuitry and associated memory circuitry, such as electronic memory 308. In such embodiments, the memory circuitry may store programs, routines, and/or encoded algorithms executed by the control processor 306 to operate the radiography imaging system 10 and the biopsy imaging device 302, including the X-ray source 16 and/or filter assembly 328 and to process the digital measurements acquired by the detector 316 in accordance with the steps and processes discussed herein. In one embodiment, the control processor 306 may be implemented as all or part of a processor-based system.

The radiation source 16 may be controlled by an X-ray controller 338 contained within the control processor 306. The X-ray controller 338 may be configured to provide power, timing signals, and/or focal size and spot locations to the radiation source 16. In addition, in some embodiments the X-ray controller 338 may be configured to selectively activate the radiation soured 16 such that tubes or emitters at different locations within the radiography imaging system 10 may be operated in synchrony with one another or independent of one another or to switch the source between different energy profiles during an imaging session.

The control processor 306 may include a data acquisition system (DAS) 340. The DAS 340 receives data collected by readout electronics of the detector 316, such as digital signals from the detector 316. The DAS 340 may then convert and/or process the data for subsequent processing by a processor-based system, such as a computer 342 operably connected to the control processor 306. In certain implementations discussed herein, circuitry within the detector 316 may convert analog signals of the detector to digital signals prior to transmission to the data acquisition system 340. The computer 342 may include or communicate with one or more non-transitory memory devices 308 that can store data processed by the computer 342, data to be processed by the computer 342, or instructions to be executed by image processing circuitry 344 of the computer 342. For example, a processor of the computer 342 may execute one or more sets of instructions stored on the memory 308, which may be a memory of the control device 304, the computer 342, or other memory of the processor, firmware, or a similar instantiation, to perform image acquisition and reconstruction techniques and/or processes.

The computer 342 may also be adapted to control features enabled by the control processor 306 (i.e., scanning operations and data acquisition), such as in response to commands and scanning parameters provided by an operator via the user interface 310 and/or operator workstation 348. Further, the display 312/350 and/or the printer 352 coupled to the control device 304 and user interface 310 allows the operator to view relevant system data, imaging parameters, raw imaging data, reconstructed data (e.g., soft tissue images, bone images, segmented vascular trees, and so on), material basis images, and/or material decomposition, and so forth. Further, the control processor 306 may include or be coupled to a picture archiving and communications system (PACS) 354. PACS 354 may be coupled to a remote system or client 356, radiology department information system (RIS), hospital information system (HIS) or to an internal or external network, so that others at different locations can gain access to the image data.

Looking now at the exemplary embodiments of FIGS. 5 and 6, the biopsy imaging device 302 includes a housing 314 formed of an x-ray transparent material and within which is disposed a photon counting energy discriminating (PCED) detector 316 that is operably connected to the control processor 306 of control device 304 and/or the controller 32 of the radiography system 10 via the wired connection 303. The housing 314 is dimensioned to support a biopsy sample holder 318 thereon in alignment with the PCED detector 316. The housing 314 is shaped as a box 315 including a number of side panels 317 extending upwardly from the surface 50 and a top panel 319 joining the side panels 317 opposite the surface 50 and forming a tissue support surface for the biopsy sample holder 318. The housing 314 can also include a bottom panel 321 opposite the top panel 319, and one or more of the side panels 317, top panel 319 and/or bottom panel 312 can be selectively removeable to allow access to the PCED 316 and other components within the interior of the housing 314. The positioning of the housing 314 on the surface 50 of the detector 18 enables the movement of the detector 18 with regard to the source 16 to additionally move the housing 314 in order to properly locate the sample holder 318 and detector 316 for proper magnification of the image to be produced of the sample(s) 320 disposed within the sample holder 318. Further, in alternative exemplary embodiments of the disclosure, the top panel 319 can be formed with the sample holder 318 integrated into the top panel 319, or with suitable attachment structures (not shown) adapted to engage and hold the sample holder 318 on the top panel 319 during operation of the biopsy imaging system 300. In still a further alternative exemplary embodiment, the housing 314 can be deployed on the surface 50 in situations where the breast 52 of the patient is still engaged under compression on the radiography system 10, such as between the compression plate 28 and the top panel 319 of the housing 314, as well as in situations where the breast 52 is not engaged under compression on the radiography system 10.

The PCED detector 316 present within the biopsy imaging device 302 provides image data to the control device 304 and/or the controller 32 for use in high resolution image generation compared to standard embedded detectors, i.e., detector 18 utilized primarily for use in producing diagnostic images. The PCED detector 316 can additionally be optimized for biopsy specimen imaging, i.e., can be configured to be application and/or subject or tissue specific. The PCED detector 316 provides enhanced resolution images, i.e., with reduced noise, and is employed in conjunction with the radiation source 16 to provide image data used to form multi-energy material decomposition images, material enhanced images, 2D/3D image reconstruction images (tomographic images), and phase contrast images.

The PCED detector 316 can have any desired configuration, such as that disclosed in US Patent No. 10,827,992, entitled *Energy-Discriminating Photon-Counting Detector And The Use Thereof,* which is expressly incorporated herein by reference in its entirety for all purposes, an provides the radiography imaging system 10 with greatly improved image resolution capabilities for producing high resolution images of the biopsy sample(s) 320 disposed within the sample holder 318, which can have any suitable x-ray transparent form, such as a plastic container, e.g., petri dish, that can be positioned on the housing 314 between the source 16 and the PCED detector 316. With the capabilities of the PCED detector 316 for detecting or measuring spectral information of the intensity or energy of the photons/x-ray radiation contacting the PCED detector 316 in energy-discrimination imaging processes (e.g., categorizing incident photons/x-ray radiation into two or more energy bins based on energy signal generated by the photons/x-ray radiation and measured by the PCED 316), the biopsy imaging device 302 in conjunction with the radiography imaging system 10 can produce various high resolution images of and/or information regarding the constituent tissue(s) in the biopsy sample(s) 320, including but not limited to, the molecular decomposition images of the biopsy sample(s) 320 and real-time information on the characteristics of tissue(s) within the biopsy sample(s) 320, malignancy, material-specific images, etc.

The PCED detector 316 can be moveably positioned or supported within the housing 314 to enable the PCED detector 316 to be moved relative to the housing 314. In the illustrated exemplary embodiment of FIG. 7, the PCED detector 316 is operably connected to a motor 324 that can displace the PCED detector 316 within the housing 314. The motor 324 is operably connected to the control device 304 and/or controller 32 in order to control the movement of the PCED detector 316 within the housing 314, optionally in connection with the movement of the radiation source 16, for operation of the biopsy imaging device 302 in a tomographic imaging process, e.g., a photon-counting computed tomography (PCCT) process.

Further, as shown in the exemplary embodiments of FIGS 5 and 7, the biopsy imaging system 300 can include a camera 370 disposed within the room (not shown) within which the radiography imaging system 10 is positioned, and in one embodiment, disposed on or within the gantry 90 spaced from the radiation source 16. The camera 370 is operably connected to the control device 304 and/or controller 32 and provides a visual indication of the position on the housing 314 of the sample holder 318 and sample(s) 320 disposed therein on the top panel 319 of the housing 314 that can be employed along with reference information, including but not limited to, the size of the housing 314 and the known location of the surface 50, to precisely determine the distance of the radiation source 16 relative to the sample(s) 320. The camera 370 can additionally provide information relating to the position of the breast 52 in situations where the biopsy imaging system 300 is operated with the breast 52 still under compression on the radiography system 10, e.g., compressed between the compression plate 28 and the top panel 319 of the housing 314. With the information provided by the camera 370 regarding the location of the sample(s) 320, the control device 304 can configure the components of the radiography imaging system 10, such as the radiation source 16 and/or the collimator 20, and/or the biopsy imaging device 302, such as the detector 316 and/or the magstand 322, to obtain the desired images of the sample(s) 320.

Further, in the illustrated exemplary embodiment of FIG. 7, a grid 326 can be positioned within the housing 314 in a position spaced from the PCED detector 316 and can be formed in exemplary embodiments as a 1D slit grid or a 2D matrix grid. The grid 326 can be present to decrease scatter in the x-rays generated by the source 16 and directed at the PCED detector 316 through the sample(s) 320. Further, optionally in conjunction with the motor 324, when present, the grid 326 can enable the operation of the biopsy imaging device 302 in a slit scan mode for imaging the biopsy sample(s) 320.

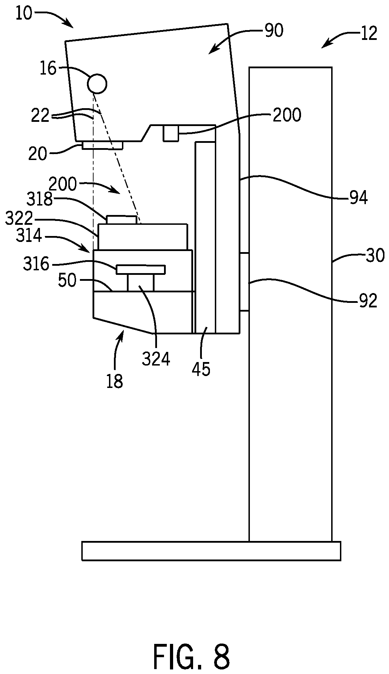

In addition, as shown in the exemplary embodiment illustrated in FIG. 8, in an alternative embodiment the housing 314 can be disposed on, or alternatively below, a magstand 322 to further elevate the sample holder 318 into the desired magnification position relative to the source 16. The magstand 322 can be a separate structure from the housing 314 or can be integrated into the form of the housing 314, such as an extendable upper portion (not shown) of the housing 314 that is operably connected to a lower portion (not shown) within which the detector 316 is positioned.

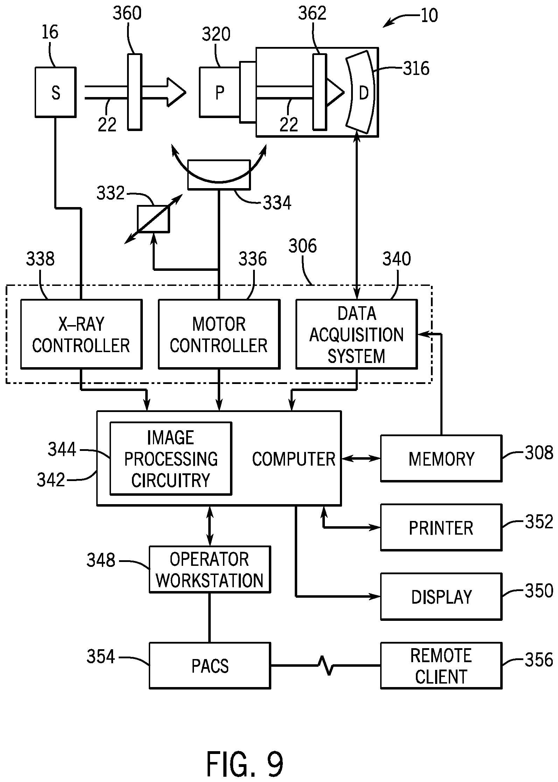

Referring now to the exemplary embodiment illustrated in FIG. 9, the biopsy device 302 can be configured to include multiple, i.e., two or more, grids 360,362 in order to obtain phase contrast images of the sample(s) 320 with the biopsy imaging device 302 and incorporated PCED detector 316, in a structure and method similar to that disclosed in US Patent No. 11,389,124, entitled *X-ray Phase Contrast Detector,* the entirety of which is expressly incorporated herein by reference for all purposes. In this embodiment for the biopsy imaging device 302, a first grid 360 is positioned between the radiation source 16 and the sample(s) 320and a second grid 362 is positioned between the sample(s) 320 and the PCED detector 316. The second grid 362 can be formed of the grid 326 disposed within the housing 314 as discussed in prior embodiments or can be a separate grid 362 located externally of the housing 314, such as a grid 362 located within a magstand 322 positioned between the housing 314 and the sample 320. With the configuration of the grids 360,362 located on either side of the sample(s) 320 and between the radiation source 16 and the PCED detector 316, the control device 304 can operate the radiography imaging system 10 to obtain phase contrast images to provide enhanced differentiation of soft tissues within the sample(s) 320 for accurate characterizations of the tissues for histopathology purposes. This phase contrast information from the PCED 316 and/or the spectral information provided by the PCED detector 316 regarding the sample(s) 320 enables the identification of the composition of the tissue(s) within the sample(s) 320 and classification of those tissue(s), e.g., as microcalcifications, cysts, solid masses, etc., with the biopsy imaging device 302 on the radiography imaging system 10.

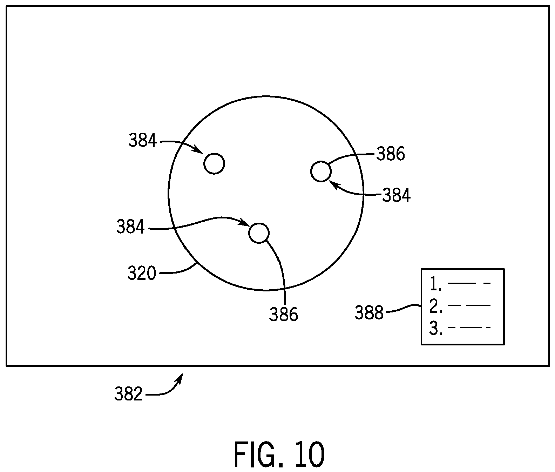

Looking now at the exemplary embodiment of FIGS. 7 and 10, to enhance the output of the biopsy imaging system 300 with regard to the classification of the tissue(s) present within the imaged sample(s) 320 based on PCED and/or phase contrast images of the sample(s) 320, the controller 32, the control processor 306, and/or the image processing circuitry 344 in the computer 342 can include an artificial intelligence AI and/or machine learning ML algorithm or module 380 trained to analyze multi-energy and phase-contrast images 382 provided by the PCED detector 316 to identify tissue types present within the sample(s) 320 and enhance visualization of suspicious tissue(s) or regions of interest (ROIs) 384 within the imaged sample(s) 320 and provide summarized information on the characteristics (e.g., probability of malignancy) of the ROIs 384. In an exemplary embodiment, after the creation of the image(s) 382 by the control processor 306 and/or image processing circuitry 344 for presentation on the display 312/350, the AI/ML model 380 analyzes the image 382 and the spectral information provided by the PCED 316 to determine the tissue type present in the image 382. This, in turn, enables the AI/ML module 380 to locate ROIs 384 within the image 382 which include, but are not limited to, various tissue types and/or structures that constitute anomalies 386, e.g., microcalcifications, cysts, solid masses, etc., or other regions of clinical relevance present within the image 382 using known identification processes and/or algorithms for x-ray imaging system image generation. For example, traditional image processing techniques, or Artificial Intelligence (AI) based-approaches including machine learning (ML) and deep learning (DL), among others, or a combination of both can be used to identify/classify and localize these ROIs 384 and anomalies 386 within the image 382. For AI based identification approaches the end goal of identifying and localizing these ROIs 384 and anomalies 386 could be formulated as either image segmentation or object localization problem. Though ML based approaches like support vector machines (SVM), random forest (RF), etc., can be used to solve these problems, convolutional neural networks (CNN), a class of DL based models, are best suited for such tasks yielding much better accuracy and adaptability across various imaging conditions. Additionally, with the identification and classification of tissues present within the biopsy sample(s) 320 by the AI/ML module 380, the AI/ML module 380 can output these results along with on-site recommendations for further review based on results illustrating malignant tissues present in the biopsy sample(s) 320, such as by providing visual indications 388 of the AI/ML module 380 assessment results in association with the image 382 of the sample(s) 320.

With respect to exemplary forms for the AI/ML module 380, a typical mammography image detection and classification algorithm can be configured as follows:
1. Examples of inputs to the AI/ML module 380: RAW/processed images (one view, or all views), multi-energy x-ray acquisitions, molecular decomposition & phase-contrast images, prior images, patient data (age, risk factors, history, ...).
2. Examples of outputs from the AI*/ML module 380: lesion location, malignancy, histoprognostic tumors makers such as estrogen and triple-negative receptors status, histological class, microcalcifications analysis.
3. Examples of AI/ML/DL models: CNN/encoders for feature extraction + dense classification layers, radiomic feature extraction, and/or combination of both, e.g., modules shown and described in:
   https://www.nature.com/articles/s41598-018-22437-z, for malignancy analysis,
   https://link.springer.com/article/10.1007/s00330-022-08538-4, for histoprognosis of tumors,
   https://link.springer.com/article/10.1007/s10278-021-00508-4, for histological class analysis, and
   https://www.mdpi.com/1660-4601/19/4/2159, for microcalcifications analysis,
each of which are expressly incorporated by reference herein in their entirety for all purposes. Further, with regard to the training of the AI/ML module 380 for one or more of these analyses, the ground truth for training (outputs) are generally provided by radiology and histopathology reports relating to the input images and patient data.

In other alternative and exemplary embodiments, the radiography imaging system 10 is not limited to a mammography imaging system 12 but can be any x-ray imaging system employed in the context of medical imaging where it is desired to obtain improved visualization of ROIs 384 in imaged tissue/biopsy sample(s) 320 using existing radiology imaging systems 10, in conjunction with an on site characterization of imaged tissue characteristics and malignancy probability, and associated triage or recommendations regarding further imaging, testing or treatment, without having to wait for off-site histopathology results. However, it should be appreciated that the present techniques are not limited to such medical contexts. Indeed, the provision of examples and explanations in such a medical context is only to facilitate explanation by providing instances of real-world implementations and applications. However, the present approaches may also be utilized in other contexts, such as the non-destructive inspection of manufactured parts or goods (i.e., quality control or quality review applications), and/or the non-invasive inspection of packages, boxes, luggage, and so forth (i.e., security or screening applications). In general, the present approaches may be desirable in any imaging or screening context in which energy discrimination in a photon-counting context is desirable.

Further, while the above description illustrates the use of the biopsy imaging system 300 for providing high resolution images, composition information and classification for biopsy sample(s) 320, the biopsy imaging system 300 can be utilized for imaging other materials and body components, including but not limited to the entire breast (not shown) or other body parts of a patient, which may be positioned on the radiography imaging system 10 in a known manner in conjunction with the biopsy imaging device 302 for use in imaging the breast or other body part in one or more of the previously described manners.

It is understood that the aforementioned compositions, apparatuses and methods of this disclosure are not limited to the particular embodiments and methodology, as these may vary. It is also understood that the terminology used herein is for the purpose of describing particular exemplary embodiments only, and is not intended to limit the scope of the present disclosure which will be limited only by the appended claims.

## Claims

1. A tissue imaging system (300) for use in conjunction with a radiography imaging system (12) including a radiation source (16) and a detector (18), the tissue imaging system (300) comprising:
a tissue imaging device (302) adapted to be positioned on a surface (50) of the detector (18) of the radiography imaging system (12) between the radiation source (16) and the detector (18), the tissue imaging device (302) comprising:
a. a housing (314) positionable on the surface (50) of the detector (18), the housing (314) defining an interior and a tissue support surface (319) opposite the surface (50) of the detector (18); and
b. a photon counting energy discriminating (PCED) detector (316) disposed within the interior of the housing (314), the PCED detector (316) adapted to generate image data used to form one or more of multi-energy material decomposition images, material enhanced images, 2D/3D image tomographic reconstruction images, and phase contrast images of the imaged tissue.

2. The tissue imaging system (300) of claim 1, further comprising a control device (304) operably connected to the PCED detector (316).

3. The tissue imaging system (300) of claim 2, wherein the control device (304) comprises an artificial intelligence module (380) configured to classify tissue types detected within the image data of the imaged tissue.

4. The tissue imaging system (300) of claim 1, wherein the PCED detector (316) is adapted to be operably connected to the radiography imaging system (10).

5. The tissue imaging system (300) of claim 1, wherein the tissue imaging device (302) further comprises a motor (324) disposed within the housing (314) and operably connected to the PCED detector (316) to move the PCED detector (316) within the interior of the housing (314).

6. The tissue imaging system (300) of claim 1, wherein the tissue imaging device (302) further comprises at least one grid (326) spaced from the PCED detector (316).

7. The tissue imaging system (300) of claim 6, wherein the at least one grid (326) is disposed within the interior of the housing (314) between the tissue support surface (319) and the PCED detector (316).

8. The tissue imaging system (300) of claim 6, further comprising:
a. a first grid (360) adapted to be positioned between the sample support surface (319) and the radiation source (16); and
b. a second grid (362) positioned between the sample support surface (319) and the detector (18).

9. The tissue imaging system (300) of claim 1, further comprising a camera (370) adapted to obtain images of the tissue support surface (319) for providing positioning information of the tissue support surface (319) and PCED detector (316) relative to the radiation source (16).

10. A radiography imaging system (10) comprising:
a. a radiation source (16);
b. a detector (18) capable of receiving radiation from the radiation source (16) and defining a surface (50);
c. a controller (32) operable to control the operation and movement of the radiation source (16) and detector (18) to generate image data, the controller (32) including a central processing unit and interconnected memory for processing the image data from the detector (18),
d. a display (38) operably connected to the controller (32) for presenting information to a user;
e. a user interface (44) operably connected to the controller (32) to enable user input to the controller (32); and
f. a tissue imaging system (300) comprising:
i. a housing (314) removably positionable on the surface (50) of the detector (18), the housing (314) defining an interior and a tissue support surface (319) opposite the surface (50) of the detector (18); and
ii. a photon counting energy discriminating (PCED) detector (316) disposed within the interior of the housing (314), the PCED detector (316) removably connected to the controller (32) and adapted to generate image data of the imaged tissue to form one or more of multi-energy material decomposition images, material enhanced images, 2D/3D image tomographic reconstruction images, and phase contrast images of the imaged tissue.

11. The radiography imaging system (10) of claim 10, further comprising a control device (304) operably connected to the PCED detector (316) including an artificial intelligence module (380) configured to classify tissue types detected within the image data of the imaged tissue.

12. The radiography imaging system (10) of claim 10, wherein the tissue imaging device (300) further comprises a motor (324) disposed within the housing (314) and operably connected to the PCED detector (316) to move the PCED detector (316) within the interior of the housing (314).

13. The radiography imaging system (10) of claim 10, wherein the tissue imaging device (300) further comprises at least one grid (326) spaced from the PCED detector (316).

14. The radiography imaging system (10) of claim 10, further comprising a magstand (322) engaged with the housing (314) to alter the position of the tissue support surface (319) relative to the surface (50) of the detector (18).

15. The radiography imaging system (10) of claim 10, further comprising a camera (370) operably adapted to obtain images of the tissue support surface (319) for providing positioning information of the tissue support surface (319) and PCED detector (316) relative to the radiation source (16).
